# EUROPEAN PATENT APPLICATION

(11) **EP 2 316 961 A1**
(43) Date of publication of application: **04.05.2011**
(21) Application number: 09773440.4
(22) Date of filing: 29.06.2009
(51) Int. Cl.: C12P 7/40, C12N 15/09, C12P 7/56

(54) **PROCESS FOR PRODUCING ORGANIC ACID**

(30) Priority: 30.06.2008 JP 2008170854
(71) Applicant: Toyota Jidosha Kabushiki Kaisha, Toyota-shi, Aichi 471-8571 (JP)
(72) Inventor: ONISHI Toru, Toyota-shi Aichi 471-8571 (JP); MATSUSHITA Hibiki, Toyota-shi Aichi 471-8571 (JP); TADA Nobuki, Toyota-shi Aichi 471-8571 (JP); ISHIDA Nobuhiro, Aichi-gun Aichi 480-1192 (JP); SHIMAMURA Takashi, Aichi-gun Aichi 480-1192 (JP)
(74) Representative: O'Brien, Simon Warwick
(86) International application number: PCT/JP2009/061863
(87) International publication number: WO 2010/001862

(57) **Abstract**

This invention is intended to improve the efficiency of organic acid fermentation in a simple manner without mutant breeding, DNA recombination breeding, or other means, when producing an organic acid via fermentation with the use of yeast that produces the organic acid of interest. The efficiency of the yeast's ability to produce organic acid is significantly improved by treating yeast that produces an organic acid in an organic-acid-containing medium having a low pH value.

SEQUENCE LISTING

<110> TOYOTA JIDOSHA KABUSHIKI KAISHA
KABUSHIKI KAISHA TOYOTA CHUO KENKYUSHO

<120> A method for producing an organic acid

<130> PH-4063-PCT

<150> JP 2008-170854
<151> 2008-06-30

<160> 10

<170> PatentIn version 3.4

<210> 1
<211> 32
<212> DNA
<213> Artificial

<220>
<223> primer

<400> 1
atatatggat ccggtagaat cattttgaat aa 32

<210> 2
<211> 32
<212> DNA
<213> Artificial

<220>
<223> primer

<400> 2
atatatgaat tctgtttatg tgtgtttatt cg 32

<210> 3
<211> 32

<212>  DNA
<213>  Artificial

<220>
<223>  primer

<400>  3
atatatgaat tcatgcatat tcaacgggaa ac                                    32


<210>  4
<211>  33
<212>  DNA
<213>  Artificial

<220>
<223>  primer

<400>  4
atatatctta agttacaacc aattaaccaa ttc                                   33


<210>  5
<211>  31
<212>  DNA
<213>  Artificial

<220>
<223>  primer

<400>  5
atatatctta agacaggccc cttttccttt g                                     31


<210>  6
<211>  30
<212>  DNA
<213>  Artificial

<220>
<223>  primer

<400> 6
atatatccgc gggttacatg cgtacacgcg 30

<210> 7
<211> 31
<212> DNA
<213> Artificial

<220>
<223> primer

<400> 7
atatatgagc tcgttggcaa tatgtttttg c 31

<210> 8
<211> 34
<212> DNA
<213> Artificial

<220>
<223> primer

<400> 8
atatatgcgg ccgcttccaa gcatctcata aacc 34

<210> 9
<211> 34
<212> DNA
<213> Artificial

<220>
<223> primer

<400> 9
atatatgggc ccgatgtcgt acgatcgcct gcac 34

<210> 10
<211> 33

<212>  DNA
<213>  Artificial

<220>
<223>  primer

<400>  10
atatatggta ccgggcaagt aacgacaaga ttg                                    33

## Description

### Technical Field

The present invention relates to a process for producing an organic acid using yeast that produces an organic acid, such as lactic acid.

### Background Art

When organic acids are produced via fermentation with the use of microorganisms, in general, fermentation is carried out by adjusting the pH value to neutral with the aid of a neutralizer. However, organic acids produced via such technique are in the form of salts, and desalting is necessary in the process of purification, which leads to an increase in the production cost. Thus, organic acids may be subjected to fermentation under acidic conditions. However, it is known that growth of acidophilic bacteria is inhibited by organic acids, such as acetic acid, lactic acid, or succinic acid *(*Kyokugen Biseibutsu (Ultimate Microorganism) Handbook, Tairo Ohshima (ed.), p. 231). Except for the case of citric acid fermentation by *Aspergillus spp.,* there are substantially no bacteria that are known to efficiently produce organic acids under acidic conditions. As techniques for overcoming such drawbacks, a method in which acid tolerance is imparted to a strain that efficiently produces an organic acid via mutant breeding (R. Patnaik et al., Nat. Biotechnol., 20, 2002, pp. 707-712) and a method of metabolic engineering aimed at improving the yield with the use of a strain with high acid tolerance but a low production capacity as a parent strain (JP Patent Publication (kohyo) No. 2003-500062 A) are disclosed, although the effects thereof are insufficient.

Thus, improvement in the ability to produce organic acids has been attempted by modifying microorganisms via mutant breeding or via genetic engineering. Such microorganism modification techniques, however, are complicated, and desired properties cannot be always attained.

### Disclosure of the Invention

### Object of the Invention

It is an object of the present invention to provide a method for improving the efficiency of organic acid fermentation in a simple manner without mutant breeding or DNA recombination breeding, when producing an organic acid via fermentation with the use of yeast that produces an organic acid of interest.

### Means for Attaining the Object

The present inventors have conducted concentrated studies in order to attain the above object. As a result, they discovered that treatment of yeast that produces an organic acid in an organic-acid-containing medium having a low pH value would significantly improve the efficiency of such yeast for organic acid production. This has led to the completion of the present invention.

Specifically, the process for producing an organic acid of the present invention comprises step "a" of treating yeast capable of producing an organic acid in an organic-acid-containing medium having a low pH value and subsequent step "b" of producing an organic acid via fermentation with the use of the above yeast.

In the process for producing an organic acid of the present invention, step "a" comprises step "c" of producing an organic acid via fermentation with the use of the above yeast, and the pH value of an organic-acid-containing medium may be adjusted at a low level with the organic acid produced in step "c." More specifically, the present invention can be applied to, for example, a continuous culture technique in which yeast is treated in an organic-acid-containing medium having the pH value adjusted at a low level with an organic acid between fermentation steps "b" and "c."

In the process for producing an organic acid of the present invention, step "c" comprises neutralizing the organic acid produced by yeast via alkaline addition during yeast fermentation. By terminating or reducing alkaline addition, the pH value of an organic-acid-containing medium may be adjusted at a low level with the organic acid produced by yeast. Specifically, an organic acid used for maintaining a low pH value may be the organic acid produced by yeast, instead of an organic acid added from outside the culture system.

In the process for producing an organic acid of the present invention, further, the organic-acid-containing medium may contain an organic acid released upon addition of an inorganic acid in a medium containing an organic acid salt. When an organic acid produced by yeast is neutralized with an alkali and contained in a medium in the form of an organic acid salt as described above, specifically, an organic acid can be released with the addition of an inorganic acid (e.g., a strong acid). Thus, an organic-acid-containing medium for yeast treatment can be prepared.

In the process for producing an organic acid of the present invention, also, the pH value of the organic-acid-containing medium may be adjusted at a low level with the addition of an organic acid from outside.

Examples of the organic acid used for maintaining a low pH value include at least one organic acid selected from the group consisting of lactic acid, succinic acid, and pyruvic acid.

In the process for producing an organic acid of the present invention, it is preferable that yeast of the genus *Saccharomyces,* and particularly yeast of the *Saccharomyces cerevisiae* strain, be used. In the process for producing an organic acid of the present invention, it is preferable that a mutant into which the lactate dehydrogenase gene has been introduced be used as the yeast. In the process for producing an organic acid of the present invention, specifically, an organic acid to be produced is preferably lactic acid.

### Effects of the Invention

According to the present invention, the capacity of yeast that produces an organic acid to produce the organic acid of interest can be improved in a very simple manner, and the ability to produce an organic acid via fermentation can be remarkably improved.

### Brief Description of the Drawings

Fig. 1 is a characteristic diagram showing the correlation between lactic acid stress application and pH value in the fermentation test.
Fig. 2 is a characteristic diagram showing changes in pH values caused by an organic acid produced by yeast in various media.
Fig. 3 is a characteristic diagram showing the results of the fermentation test when pH stress is applied by an organic acid produced by yeast. The concentration (%) indicated on the vertical axis is the concentration of a fermented mash composition 18 hours after the initiation of fermentation.

### Best Modes for Carrying out the Invention

This description includes part or all of the contents as disclosed in the description and/or drawings of Japanese Patent Application No. 2008-170854, which is a priority document of the present application.

Hereafter, the present invention is described in detail.

In the present invention, yeast that produces an organic acid is treated in an organic-acid-containing medium having a low pH value prior to fermentative production of an target organic acid. Yeast that produces an organic acid may be yeast that is naturally capable of organic acid production or yeast that has attained the capacity for organic acid production via genetic engineering or other means. Examples of yeast include yeast of the genus *Saccharomyces,* yeast of the genus *Shizosaccharomyces,* yeast of the genus *Candida,* yeast of the genus *Pichia,* yeast of the genus *Hansenula,* yeast of the genus *Torulopsis,* yeast of the genus *Yarrowia,* yeast of the genus *Kluyveromyces,* yeast of the genus *Zygosaccharomyces,* yeast of the genus *Yarrowia,* and yeast of the genus *Issatchenkia.* Use of a *Saccharomyces cerevisiae* yeast strain is particularly preferable. Further, use of a *Saccharomyces cerevisiae* mutant into which a single copy or a plurality of copies of the lactate dehydrogenase (LDH) gene have been introduced that has attained the capacity for lactic acid production is preferable. An organic acid produced by yeast is preferably lactic acid, although an organic acid is not limited thereto. For example, organic acids, such as pyruvic acid, succinic acid, citric acid, fumaric acid, malic acid, acetic acid, 3-hydroxypropionic acid, malonic acid, propionic acid, aspartic acid, glutamic acid, itaconic acid, levulinic acid, ascorbic acid, and gluconic acid, can be the targets of production.

It is necessary that a wild-type yeast strain that produces ethanol as a major metabolite be subjected to metabolic modification in order to attain the capacity for organic acid production. When providing a yeast strain with the capacity for lactic acid production, for example, a gene associated with lactic acid biosynthesis may be transformed.

Examples of genes associated with lactic acid biosynthesis include a lactate dehydrogenase gene disclosed in JP Patent Publication (kokai) No. 2003-259878 A, a lactic bacteria-derived gene (J. Ind. Microbiol. Biotechnol., 31, 209-215, JP Patent Publication (kohyo) No. 2005-518197 A), a *Bacillus megaterium-derived* gene (JP Patent Publication (kohyo) No. 2005-518197 A), a mold *(Rhizopus)*-derived gene (J. Ind. Microbiol. Biotechnol., 30, 22-275), and a cattle-derived gene (Appl. Environ. Microbiol., 67, 5621-5625). Examples of other genes that can be used include genes of lactate dehydrogenase derived from procaryotes such as lactic bacteria, eucaryotes such as molds, and higher eucaryotes such as plants, animals, and insects.

Use of a yeast strain into which the lactate dehydrogenase gene has been introduced (disclosed in JP Patent Publication (kokai) No. 2003-259878 A) as yeast capable of lactic acid production is particularly preferable. Since expression of the lactate dehydrogenase gene is optimized in yeast, L-lactic acid can be produced with high efficiency upon transformation into yeast. An example of a transformed microorganism is a recombinant yeast strain (Appl. Environ. Microbiol., 1999, 65 (9); 4211-4215).

Such yeast is treated via contact with an organic-acid-containing medium having a low pH value. The term "low pH value" used herein refers that a medium is under acidic conditions. At a low pH value, specifically, the pH is lower than 7.0, preferably 4.0 or lower, and more preferably 3.5 or lower. In the case of *Saccharomyces cerevisiae,* the optimal pH for culture and fermentation is between 5.0 and 6.0 for ehtanol fermentation. When culture and fermentation are carried out at the pH of 4.0 or lower, growth and the ability of strains to produce ethanol are significantly inhibited. Some target yeast strains to be treated may die under strong acidic conditions. Accordingly, it is preferable that the pH value be within a range that would not cause the target yeast strains to die. For example, it is preferable that the lower limit of the pH value of a medium used for treating a *Saccharomyces cerevisiae* mutant into which the LDH gene has been introduced be 2.0.

A medium in which yeast is treated contains an organic acid, and the pH value thereof is low. An organic acid used for maintaining a low pH value is at least one type of organic acid selected from the group consisting of lactic acid, succinic acid, and pyruvic acid. Organic acids are not limited thereto, and examples of organic acids include acetic acid, formic acid, benzoic acid, citric acid, D-glucuronic acid, oxalic acid, fumaric acid, malic acid, 3-hydroxypropionic acid, malonic acid, propionic acid, aspartic acid, glutamic acid, itaconic acid, levulinic acid, ascorbic acid, and gluconic acid.

An organic-acid-containing medium may be prepared by adding the aforementioned organic acid to the medium composition described below from outside. Alternatively, an organic-acid-containing medium may be prepared by adding a strong acid such as sulfuric acid to an organic-acid-salt-containing composition and releasing the organic acid. Any acid may be used for releasing an organic acid from the organic acid salt without particular limitation, provided that such acid is stronger than the target organic acid.

The composition of a medium used for yeast treatment is not particularly limited. For example, any conventional assimilable carbon sources of yeast capable of organic acid production can be used as assimilable carbon sources for a fermentation medium. As long as yeast can grow and produce organic acid, therefore, it may adequately selected in accordance with the type of yeast to be used. Examples of assimilable carbon sources that can be used include glucose, maltose, sucrose, molasses, corn steep liquor, and saccharides from cellulosic materials. Examples of nitrogen sources for fermentation medium that can be used include a yeast extract, peptone, and whey. In order to prepare a cost-effective medium that would not disturb the process of purification, it is preferable that a nitrogen source not be added and inorganic nitrogen from an ammonium salt such as ammonium sulfate or urea be used. In addition, potassium phosphate, magnesium sulfate, Fe (iron), an Mn (manganese) compound, or the like can be used as an inorganic nutrient source, although such substance is not essential.

When treating the above yeast in a medium having a low pH value, treatment is preferably continued for 2 hours or longer when the pH value of a medium is adjusted to 3.0 with, for example, lactic acid. If the pH value of the medium is lower, the duration of treatment can be shortened. Treatment temperature varies depending on yeast type, and it is generally about 20°C to 40°C. Treatment may be carried out at a temperature higher than 40°C depending on yeast type. The yeast may be treated via agitation or shaking of a medium having a low pH value.

By treating yeast capable of organic acid production with a medium having a low pH value, accordingly, the capacity of the yeast for organic acid production can be improved, and acid tolerance of the yeast can further be improved. The capacity for organic acid production can be evaluated by comparing the concentration of organic acid contained in a medium used when organic acid was prepared with the use of untreated yeast with the concentration of organic acid contained in a medium used when the treated yeast was used.

Organic acid may be produced with the use of yeast via batch culture, continuous culture, or semibatch culture. Batch culture is carried out by preparing a fresh medium for each cycle of a plurality of culture cycles and inoculating the medium with yeast without the addition of a medium until the completion of organic acid production. Continuous culture is also referred to as "perfusion culture," and it is carried out by supplying a medium to a culture system at a given rate while extracting the same amount of the culture solution. Semibatch culture is carried out by continuously or intermittently adding a medium or a given component of a medium during culture. In any such culture system, the yeast may be used while being supported on an immobilization carrier.

In any such culture system, the treatment with the use of an organic-acid-containing medium having a low pH value described above may be carried out prior to the initiation of organic acid fermentative production with the use of yeast. This can significantly improve the efficiency of organic acid production via fermentation. Since a shift in pH value to acidic conditions resulting from the organic acid produced during culture is prevented in any such culture system, a neutralizing agent, such as ammonia, Ca(OH)₂, CaCO₃, or NaOH, may be added (i.e., alkaline addition). Since the acid tolerance of yeast treated in a medium having a low pH value has improved, organic acid fermentative production can be carried out without maintaining the pH value in a neutral region with the use of a neutralizing agent.

In any such culture system, the pH value of the medium may be adjusted at a low level with the aid of an organic acid at the completion of organic acid fermentative production. Thus, the capacity of the yeast for organic acid production can be improved in the subsequent organic acid fermentative production. Specifically, the process for producing an organic acid of the present invention can be applied to a production method involving a plurality of cycles of organic acid fermentative production. The capacity of the yeast for organic acid production can be improved by maintaining a low pH value at the completion of each fermentation production cycle.

When neutralizing a medium via alkaline addition at the time of organic acid fermentative production, alkaline addition may be terminated, or the amount of alkali added may be reduced to lower the pH value. Thus, an organic-acid-containing medium having a low pH value can be prepared. When an organic acid salt is incorporated into a medium via alkaline addition, an organic acid can be released in the medium with the addition of a strong acid such as sulfuric acid. Thus, an organic-acid-containing medium having a low pH value can be prepared. An organic acid can be released from an organic acid salt with the use of any acid without particular limitation, provided that it is stronger than the target organic acid.

When an organic acid is produced via fermentation with the use of the yeast, fermentative production is carried out at about 20°C to 40°C, although the temperature conditions are not particularly limited. Fermentative production can be carried out at a higher temperature depending on the type of yeast to be used. The reaction time required for organic acid production is not particularly limited, and the reaction is carried out for an arbitrary length of time, as long as the effects of the present invention are attained. Since the reaction time required for organic acid production varies inversely with respect to the amount of strains to be sown, the reaction time can be adjusted by adequately determining such amount. A person skilled in the art would readily optimize such conditions.

### Examples

Hereafter, the present invention is described in greater detail with reference to the examples, although the technical scope of the present invention is not limited to the examples below.

### [Example 1]

In Example 1, a strain into which 6 copies of lactate dehydrogenase genes had been introduced was prepared as a lactic acid-producing yeast strain with the use of the strain prepared via introduction of 4 copies of lactate dehydrogenase genes as a parent strain (such strain having been prepared by JP Patent Publication (kokai) No. 2006-006271 A) and introduction of 2 additional copies of the lactate dehydrogenase genes thereinto.

### (Construction of G418-tolerant marker cassette)

Genomic DNA of the NBRC2260 yeast strain was used as a template and a DNA fragment of the TDH3 promoter region was amplified via PCR. As PCR primers, TDH3P-U (5'-ATA TAT GGA TCC GGT AGA ATC ATT TTG AAT AA-3' (SEQ ID NO: 1); prepared with the addition of the *Bam*HI site to the TDH3 promoter sequence) and TDH3P-D (5'-ATA TAT GAA TTC TGT TTA TGT GTG TTT ATT CG -3' (SEQ ID NO: 1); prepared with the addition of the *Eco*RI site to the TDH3 promoter sequence) were used. The amplified TDH3 promoter sequence was digested with the *Bam*HI and *Eco*RI restriction enzyme, and the resultant was designated as the TDH3P fragment.

Genomic DNA of the *E*. *coli* K-12 strain was used as a template and the G418-tolerant gene fragment was amplified via PCR. As PCR primers, G418ORF-U (5'-ATA TAT GAA TTC ATG CAT ATT CAA CGG GAA AC -3' (SEQ ID NO: 3); prepared with the addition of the *Eco*RI restriction enzyme site to the G418-tolerant gene sequence) and G418ORF-D (5'-ATA TAT CTT AAG TTA CAA CCA ATT AAC CAA TTC-3' (SEQ ID NO: 4); prepared with the addition of the *Afl*II restriction enzyme site to the G418-tolerant gene sequence) were used. The amplified G418-tolerant gene sequence was digested with the *Eco*RI and *Afl*II restriction enzymes, and the resultant was designated as the G418 fragment.

Genomic DNA of the NBRC2260 yeast strain was used as a template and the DNA fragment of the CYC1 terminator region was amplified via PCR. As PCR primers, CYC1T-U (5'-ATA TAT CTT AAG ACA GGC CCC TTT TCC TTT G-3' (SEQ ID NO: 5); prepared with the addition of the *Afl*II site to the CYC1 terminator sequence) and CYC1T-D (5'ATA TAT CCG CGG GTT ACA TGC GTA CAC GCG -3' (SEQ ID NO: 6); prepared with the addition of the *Sac*II site to the CYC1 terminator sequence) were used. The amplified CYC1 terminator sequence was digested with the *Afl*II and SacII restriction enzymes, and the resultant was designated as the CYC1T fragment.

The TDH3P fragment, the G418 fragment, and the CYC1T fragment obtained by the above procedure were successively ligated in that order to the multicloning site of the pBluescriptII SK+ vector so as to align such fragments. Thus, a G418-tolerant marker cassette was constructed. The resulting vector was digested with the *SacI* and *Eco*RV restriction enzymes, the G418-tolerant marker cassette was cleaved and blunt-ended via treatment with a terminal-modifying enzyme (i.e., T4 DNA polymerase), and the resultant was designated as the G418-tolerant marker cassette fragment.

### (Construction of the pBG418-LDHKCB chromosome-introducing vector)

A chromosome-introducing vector used for introducing the LDH gene into a site between the PDC6 gene and the CTT1 gene of chromosome 7 was prepared in the following manner.

Genomic DNA of the NBRC2260 yeast strain was used as a template and a DNA fragment inthea 5' upstream region of the PDC6 gene was amplified via PCR. As PCR primers, PDC6-U (5'-ATA TAT GAG CTC GTT GGC AAT ATG TTT TTG C-3' (SEQ ID NO: 7); prepared with the addition of the *SacI* site to the 5' upstream region of PDC6) and PDC6-D (5'-ATA TAT GCG GCC GCT TCC AAG CAT CTC ATA AAC C-3' (SEQ ID NO: 8); prepared with the addition of the *Not*I site to the 5' upstream region of PDC6) were used. The amplified 5' upstream region of the PDC6 gene was digested with the *SacI* and NotI restriction enzymes, and the resultant was designated as the PDC6 fragment.

Genomic DNA of the NBRC2260 yeast strain was used as a template and the DNA fragment in the 5' upstream region of the CTT1 gene was amplified via PCR. As PCR primers, CTT1-U (5'-ATA TAT GGG CCC GAT GTC GTA CGA TCG CCT GCA C-3' (SEQ ID NO: 9); prepared with the addition of the *Apa*I site to the 5' upstream region of CTT1) and CTT1-D (5'-ATA TAT GGT ACC GGG CAA GTA ACG ACA AGA TTG-3' (SEQ ID NO: 10); prepared with the addition of the *Kpn*I site to the 5' upstream region of CTT1) were used. The amplified 5' upstream region of the CTT1 gene was digested with the *Apa*I and *Kpn*I restriction enzymes, and the resultant was designated as the CTT1 fragment.

The pBTrp-PDC1-LDHKCB plasmid prepared by JP Patent Application No. 2002-65879 (disclosed in JP Patent Publication (kokai) No. 2003-259878 A) was digested with *Bam*HI and *Pst*I*,* the cleaved fragment was designated as the LDHKCB expression cassette fragment (the fragment comprising the PDC1 promoter, the LDH gene, and TDH3 terminator ligated in that order). The fragments obtained above (i.e., the PDC6 fragment, the LDHKCB expression cassette fragment, the G418-tolerant marker cassette fragment, and the CTT1 fragment) were succssively ligated to the multicloning site of the pBluescriptII SK+ vector to construct a chromosome-introducing vector (pBG418-LDHKCB).

### (Preparation of strain comprising 6 copies of LDH that had been introduced therein)

The pG418-LDHKCB vector was digested with the *SacI* and *Kpn*I restriction enzymes by the lithium acetate method (Ito et al., J. Bacteriol., 153, 163-168, 1983), and the resulting fragment was used to transform the strain into which 4 copies of PDC1p-LDH had been introduced (prepared in JP Patent Publication (kokai) No. 2006-006271 A). Selection was carried out in a YPD medium containing G418 at 10 µg/ml, and the introduced genes were confirmed via PCR to obtain transformants.

Spores were generated from the strains in a sporulation medium (containing 1% potassium phosphate, 0.1% yeast extract, 0.05% glucose, and 2% agar), and diploidization was carried out with the utilization of homothallic properties. A strain into which the target genes had been introduced at both diploid chromosomes was obtained and designated as the strain into which 6 copies of PDC1p-LDH had been introduced.

### (Lactic acid stress application and fermentation test)

The strain into which 6 copies of PDC1p-LDH had been introduced was sown in a YPD medium containing 0.1% calcium carbonate (1% yeast extract, 2% peptone, and 2% glucose), culture was conducted at 150 rpm/min (shaking width: 35 mm) at 30°C for 22 hours, lactic acid, succinic acid, or pyruvic acid was added thereto to a given concentration, the pH value was measured, and culture was conducted for an additional 5 hours.

A fermentation medium (20 ml; concentration: 12% sugar, 0.04% potassium dihydrogen phosphate, 0.04% magnesium sulfate, and 0.4% calcium carbonate) was introduced into a 100-ml flask, the above strains were inoculated thereto to a concentration of 4%, fermentation was carried out at 120 rpm/min (shaking width: 35 mm) at 34°C, and the amount of lactic acid produced was inspected. All sugars were used up in all treatment regions or the lactic acid concentration began to fall in regions in which sugar still remained 16 hours after the initiation of fermentation. Thus, the fermentation test was completed.

Lactic acid was assayed with the use of BF-4 and BF-5 biosensors (Oji Scientific Instruments). The results of lactic acid assay and the results of assay of the pH value of the medium after acid treatment and of the pH value of the medium after the completion of culture are shown in Table 1.

**Table 1**

| | Without addition | Lactic acid | | | Succinic acid | | | Pyruvic acid |
|---|---|---|---|---|---|---|---|---|
| | | 200 mM | 400 mM | 600 mM | 200 mM | 400 mM | 600 mM | 100 mM |
| Maximal lactic acid concentration (%) | 6.0 | 5.8 | 6.5 | 7.2 | 5.0 | 6.7 | 7.2 | 6.5 |
| pH of medium after acid treatment | 4.1 | 3.5 | 3.2 | 3.1 | 3.9 | 3.8 | 3.6 | 3.2 |
| pH of medium after completion of culture completion of culture | 4.3 | 3.6 | 3.3 | 2.8 | 4.0 | 3.8 | 3.7 | 3.2 |
| Lactic acid concentration after completion of culture (%) | 0.62 | 2.15 | 3.75 | 5.09 | 0.80 | 0.55 | 0.5 | 0.80 |

As is apparent from Table 1, the results of the fermentation test demonstrate that the lactic acid concentration would be improved in regions treated with lactic acid at a concentration of 400 mM or higher and with succinic acid at a concentration of 400 mM or higher. In addition, pyruvic acid having a higher capacity for acidifying a medium at a low concentration was found to improve the lactic acid concentration at a concentration of 100 mM.

### (Lactic acid stress application and pH value in fermentation test)

The strain into which 6 copies of PDC1p-LDH had been introduced was sown in a YPD medium containing 0.1% calcium carbonate (1% yeast extract, 2% peptone, and 2% glucose), culture was conducted at 150 rpm/min (shaking width: 35 mm) at 30°C for 22 hours, the culture product was treated with 600 mM lactic acid, and culture was conducted for an additional 5 hours. The strain that had been cultured for 27 hours without lactic acid treatment was designated as a control.

The pH value of the fermentation medium was altered by varying the calcium carbonate concentration, 20 ml of such medium (concentration: 12% sugar, 0.04% potassium dihydrogen phosphate, 0.04% magnesium sulfate, and 0.1%, 0.5%, 1.0%, 2.0%, 4.0%, or 5.0% calcium carbonate) was introduced into a 100-ml flask, the two above types of cultured strains were sown therein to a concentration of 4%, fermentation was carried out at 120 rpm/min (shaking width: 35 mm) at 34°C, and the amount of lactic acid produced was inspected. The fermentation test was completed 16 hours after the initiation of fermentation. All sugars were used up 16 hours after the initiation of fermentation except for the region treated with 0.1 % calcium carbonate.

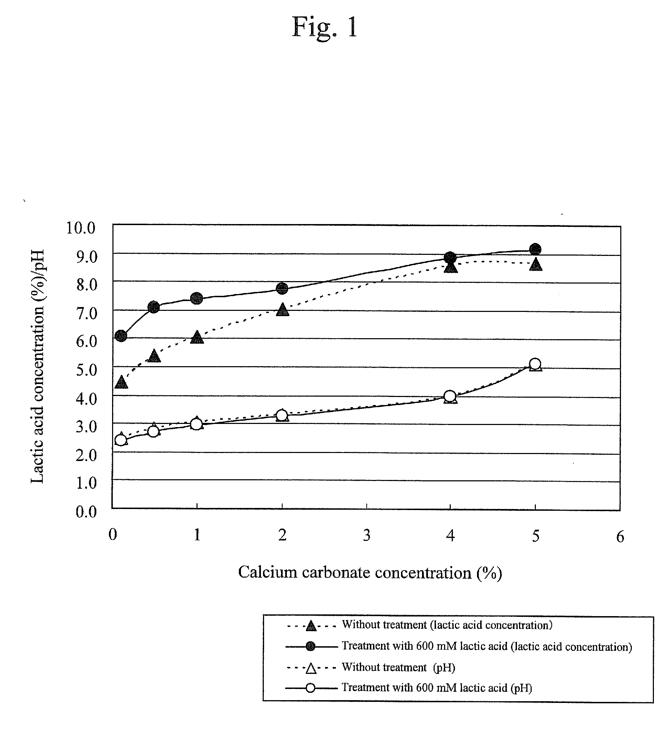

Lactic acid was assayed with the use of BF-4 and BF-5 biosensors (Oji Scientific Instruments). The results are shown in Fig. 1. In Fig. 1, the lactic acid concentration indicates the maximal concentration up to 16 hours after the initiation of fermentation, and the pH value was attained at the maximal lactic acid concentration.

As a result of the fermentation test, the strains that had been treated with 600 mM lactic acid were found to exhibit higher lactic acid concentrations in all treated regions than strains that were not treated. The pH value of the fermented mash was between 2.4 and 5.1. The results demonstrate that the effects of improving fermentation capacity via organic acid stress application are not correlated with the regulated pH value at the time of fermentation.

### (pH stress application by organic acid produced by strain and fermentation test)

Whether or not a procedure of reducing medium additives having high buffering capacity, such as yeast extract and peptone, from a culture medium and lowering of the pH value of the culture medium using only an organic acid produced by a strain would produce effects similar to those attained by lowering the pH value with the addition of an organic acid from ouside was examined. The strain into which 6 copies of PDC1p-LDH had been introduced was sown in a modified YPD medium (0.5% yeast extract, 1% peptone, and 3% glucose), and culture was conducted at 150 rpm/min (shaking width: 35 mm) at 30°C for 33 hours. As control samples, a treatment group was prepared by adding calcium carbonate 8, 24, and 29 hours after the initiation of culture to suppress the lowering of the pH value of the culture medium, and another treatment group was prepared via culture in a YPD medium without the addition of calcium carbonate.

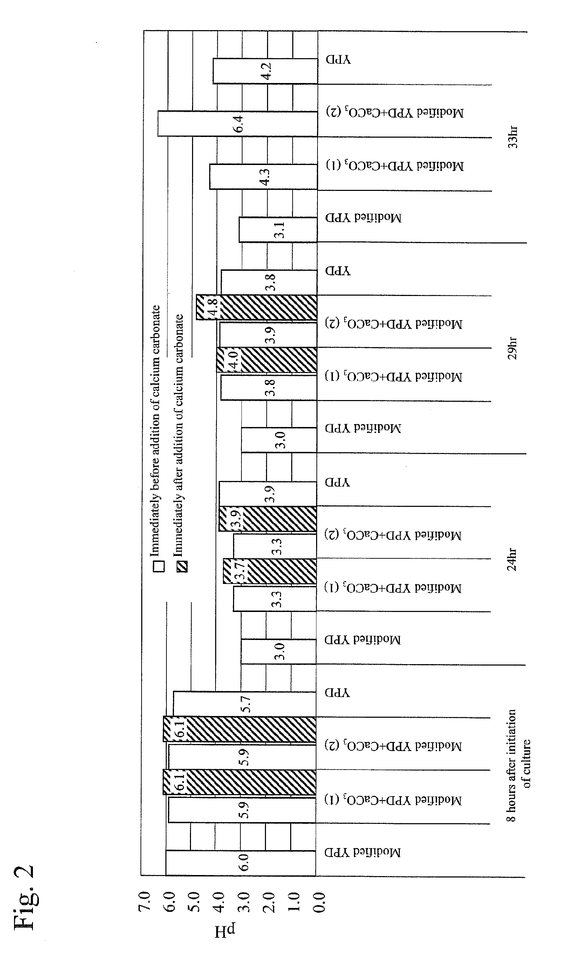

The pH value of the strain cultured in a modified YPD medium was lowered to 3.0, a treatment group (1) prepared by adding calcium carbonate to a modified YPD medium (0.1%, 0.2%, and 0.1% calcium carbonate was added 8, 24, and 29 hours later) exhibited the pH value that was lowered to 3.3 24 hours later, but the pH value became 4.3 at the completion of culture. The pH value of the treatment group (2) prepared by adding calcium carbonate to a modified YPD medium (0.1%, 0.3%, and 0.2% calcium carbonate was added 8, 24, and 29 hours later) was 6.4 at the completion of culture. When culturing took place in a YPD medium, the pH value was lowered to 3.8, but the pH value became 4.2 at the completion of culture (Fig. 2). When the lactic acid concentration in a medium at the completion of culture was assayed, lactic acid was detected. Thus, it was considered that the pH value would be lowered mainly by lactic acid (see Table 2).

**Table 2**

| | Modified YPD | Modified YPD + CaCO₃ (1) | Modified YPD + CaCO₃ (2) | YPD |
|---|---|---|---|---|
| Lactic acid concentration at the completion of culture | 0.92 | 1.11 | 1.13 | 0.40 |

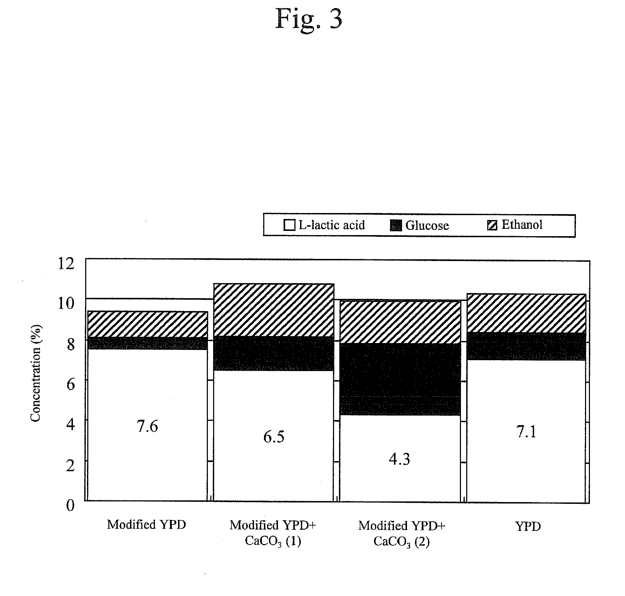

A fermentation medium (20 ml; concentration: 13.8% sugar, 0.5% calcium carbonate, 0.04% potassium dihydrogen phosphate, and 0.04% magnesium sulfate) was introduced into a 100-ml baffled flask, the above cultured strains were sown therein to a concentration of 4%, fermentation was carried out at 120 rpm/min (shaking width: 35 mm) at 34°C, and the composition of the fermented mash 18 hours later was inspected (Fig. 3). As a result of the fermentation test, the strains cultured in a modified YPD medium were found to exhibit the highest lactic acid concentrations. Lactic acid, glucose, and ethanol were assayed with the use of BF-4 and BF-5 biosensors (Oji Scientific Instruments). Further, the viable count was assayed 18 hours after the initiation of fermentation, the cell count was found to be about 30% to 50% that at the initiation of culture, and no correlations were found between the capacity for lactic acid fermentation and the viable count. The viable count was assayed with the use of an automated cell viability analyzer (Vi-Cell, Beckman Coulter Inc.).

The results demonstrate that the presence of an organic acid at a given concentration in a medium is sufficient to produce the organic acid stress that is necessary for improvement of fermentation capacity, there is no need of the addition of an organic acid from outside to maintain a low pH value, and the effects thereof vary depending on pH value.

### [Example 2]

In Example 2, the duration of treatment of the strain into which 6 copies of PDC1p-LDH had been introduced used in Example 1 in an organic acid was inspected. Specifically, the strain into which 6 copies of PDC1p-LDH had been introduced was sown in a YPD medium containing 0.1 % calcium carbonate (1% yeast extract, 2% peptone, and 2% glucose), and culture was conducted at 150 rpm/min. (shaking width: 35 mm) at 30°C for 21 hours. Thereafter, 600 mM lactic acid was added as the organic acid treatment, and culture was carried out for an additional 30 minutes to 5 hours. A strain that had been cultured for 21 hours without lactic acid treatment was designated as a control.

Subsequently, 20 ml of a fermentation medium (concentration: 13% sugar, 0.04% potassium dihydrogen phosphate, 0.04% magnesium sulfate, and 0.4% calcium carbonate) was introduced into a 100-ml flask, the above strains were sown therein to a concentration of 4%, fermentation was carried out at 120 rpm/min (shaking width: 35 mm) at 34°C, and the amount of lactic acid produced was inspected. The results of assay of lactic acid concentration and glucose concentration attained 17 hours after the initiation of fermentation are shown in Table 3. Lactic acid concentration was not elevated even when fermentation was continued for 17 hours or longer. Lactic acid and glucose were assayed with the use of BF-4 and BF-5 biosensors (Oji Scientific Instruments).

**Table 3**

| | Duration of lactic acid treatment | | | | | | |
|---|---|---|---|---|---|---|---|
| | Without treatment | 0.5 hours | 1 hour | 2 hours | 3 hours | 4 hours | 5 hours |
| Lactic acid concentration (%) | 4.4 | 4.7 | 4.6 | 5.4 | 5.7 | 5.7 | 5.8 |
| Glucose concentration (%) | 2.6 | 1.2 | 1.2 | 0.4 | 0.4 | 0.2 | 0.1 |
| Medium pH at the completion of culture | 4.1 | 3.0 | 3.0 | 3.0 | 3.0 | 3.0 | 3.0 |

As is apparent from Table 3, the results of the fermentation test demonstrate that the lactic acid concentration was significantly elevated in a region that had been treated with lactic acid for 2 hours or longer in comparison with the group that had not been treated. This indicates that the duration necessary for organic acid stress application is preferably 2 hours or longer. In Example 2, lactic acid is used as an organic acid and evaluation is carried out with the experimentation system in which the pH of a medium is 3.0 at the completion of culture. It should be thus understood that the duration necessary for organic acid application varies depending on the type of organic acid or the pH value at the completion of culture.

### [Example 3]

In Example 3, the preferable pH value for organic acid treatment of the strains into which 6 copies of PDC1p-LDH had been introduced used in Example 1 was examined. Specifically, the pH value of the strains into which 6 copies of PDC1p-LDH had been introduced was adjusted to 5.2, 4.5, 4.0, or 3.5 with the use of a 1-liter jar (Biott Co., Ltd.) and the strains were multiplied. The fermentation test was carried out with the use of strains treated at various pH values, and the amount of lactic acid produced was measured in order to inspect the influence of pH value at the time of lactic acid stress application on the fermentation test. The strains were multiplied in a 1-liter jar (i.e., lactic acid stress application) in a YPD medium, and culture was conducted at 450 rpm, an aeration rate of 1 vvm (volume per volume per minute, i.e., volume of gas flow per unit volume per minute), and 30°C for 17 hours. pH control was carried out with lactic acid and sodium hydroxide.

A fermentation medium (20 ml; 13% glucose, 0.01% potassium dihydrogen phosphate, 0.01% magnesium sulfate, and 0.4% calcium carbonate) was introduced into a 100-ml flask, the above strains were sown therein to a concentration of 1%, fermentation was carried out at 120 rpm (shaking width: 35 mm) at 34°C, and the amount of lactic acid produced was examined.

Lactic acid was assayed with the use of BF-4 and BF-5 biosensors (Oji Scientific Instruments). The results are shown in Table 4. In Fig. 4, the lactic acid concentration indicates the maximal concentration up to 54 hours after the initiation of fermentation.

**Table 4**

| | | | | | |
|---|---|---|---|---|---|
| Controlled pH at the time of multiplication of strains | 5.2 | 4.5 | 4.0 | 3.5 | 3.0 |
| Maximal lactic acid concentration | 6.4 | 6.8 | 6.8 | 6.7 | 7.2 |

The results of the fermentation test demonstrate that the maximal lactic acid concentration tends to be elevated as the pH value of a medium is lowered when strains are multiplied. Such effects were observed at pH 4.5, and the maximal effects were attained at pH 3.0.

### [Comparative Example 1]

In Comparative Example 1, the fermentation test was carried out in the same manner as in Example 1, except that an inorganic acid was used instead of an organic acid and a medium having a low pH value or a medium comprising an organic acid salt at the same molar concentration as the concentration which was effective with the use of an organic acid (with the pH value of such medium not being low). In this comparative example, specifically, sulfuric acid was added to a concentration of 20 mM or 40 mM as the inorganic acid, the pH value was measured, and culture was conducted for an additional 5 hours. The strains into which 6 copies of PDC1p-LDH had been introduced were treated in a medium having the pH value lowered with the aid of sulfuric acid. Subsequently, 20 ml of a fermentation medium (concentration: 12% sugar, 0.04% potassium dihydrogen phosphate, 0.04% magnesium sulfate, and 0.4% calcium carbonate) was introduced into a 100-ml flask, the above strains were sown therein to a concentration of 4%, fermentation was carried out at 120 rpm/min (shaking width: 35 mm) at 34°C, and the amount of lactic acid produced was examined. All sugars were used up in all treatment regions or the lactic acid concentration began to fall in regions in which sugar still remained 16 hours after the initiation of fermentation. Thus, the fermentation test was completed. Lactic acid was assayed with the use of BF-4 and BF-5 biosensors (Oji Scientific Instruments). The results are shown in Table. 5.

**Table 5**

| | Sulfuric acid | | Sodium lactate |
|---|---|---|---|
| | 20 mM | 40 mM | 600 mM |
| Maximal lactic acid concentration (%) | 3.0 | 3.2 | 5.2 |
| pH of medium after acid treatment | 3.7 | 3.0 | 5.2 |
| pH of medium after completion of culture | 3.7 | 2.9 | 5.4 |
| Lactic acid concentration after completion of culture | 0.25 | 0.41 | 4.63 |

A comparison of Table 1 with Table 5 demonstrates that treatment in a medium having a low pH value with an organic acid improves lactic acid productivity. By performing the treatment in a medium that has had its pH value lowered by an inorganic acid or a medium containing an organic acid salt the pH value of which has not been lowered, the ability to produce lactic acid was lowered. This comparative example demonstrates that treatment of yeast that produces an organic acid with an organic-acid-containing medium having a low pH value improves the capacity of such yeast to produce organic acid.

All publications, patents, and patent applications cited herein are incorporated herein by reference in their entirety.

## Claims

1. A process for producing an organic acid comprising:
step "a" of treating yeast capable of producing an organic acid in an organic-acid-containing medium having a low pH value; and
subsequent step "b" of producing an organic acid via fermentation with the use of the yeast.

2. The process for producing an organic acid according to claim 1, wherein step "a" comprises step "c" of producing an organic acid via fermentation with the use of the yeast, and the pH value of the organic-acid-containing medium is adjusted at a low level with the organic acid produced in step "c."

3. The process for producing an organic acid according to claim 2, wherein step "c" comprises neutralizing the organic acid produced by yeast via alkaline addition during fermentation with the use of yeast, with alkaline addition being terminated or reduced to maintain a low pH value for the organic-acid-containing medium with the organic acid produced by yeast.

4. The process for producing an organic acid according to claim 1, wherein the organic acid contained in the organic-acid-containing medium is added from outside.

5. The process for producing an organic acid according to claim 1, wherein the organic acid contained in the organic-acid-containing medium is at least one organic acid selected from the group consisting of lactic acid, succinic acid, and pyruvic acid.

6. The process for producing an organic acid according to claim 1, wherein the organic-acid-containing medium comprises an organic acid released via the addition of an inorganic acid in a medium containing an organic acid salt.

7. The process for producing an organic acid according to claim 1, wherein the yeast belongs to the genus *Saccharomyces.*

8. The process for producing an organic acid according to claim 1, wherein the yeast is of a *Saccharomyces cerevisiae* strain.

9. The process for producing an organic acid according to claim 1, wherein the yeast is a mutant into which the lactate dehydrogenase gene has been introduced.

10. The process for producing an organic acid according to claim 1, wherein the organic acid produced by yeast is lactic acid.
